# EUROPEAN PATENT APPLICATION

(11) **EP 4 176 824 A1**
(43) Date of publication of application: **10.05.2023**
(21) Application number: 22202378.0
(22) Date of filing: 19.10.2022
(51) Int. Cl.: A61B 17/04

(54) **KNOTLESS ANCHOR DRIVE SYSTEM**

(30) Priority: 04.11.2021 US 202163275661 P
(71) Applicant: Smith & Nephew Asia Pacific Pte. Limited, Singapore 138565 (SG); Smith & Nephew Orthopaedics AG, 6300 Zug (CH); Smith & Nephew, Inc., Memphis, TN 38116 (US)
(72) Inventor: ROGERS, Jon-Paul, Memphis, 38116 (US); HAMILTON, Jason, Memphis, 38116 (US)
(74) Representative: Appleyard Lees IP LLP

(57) **Abstract**

A suture anchor assembly is disclosed herein. The assembly includes an anchor body for anchoring within a target tissue, the anchor body including a proximal connecting portion. The assembly also includes an insertion instrument for inserting the anchor body within the target tissue. The insertion instrument includes a connecting portion at a distal end thereof that interdigitates with the anchor body connecting portion to provide rotational stability between the anchor body and insertion instrument. The connecting portions, when assembled, forms a tubular shape with a shared inner diameter and a shared outer diameter. The shared inner diameter defines a cannulation that received a plug therethrough. The plug may axially advance into the anchor body, to knotlessly lock a suture of the suture anchor assembly.

## Description

### FIELD

The present disclosure relates to a low-profile knotless anchor drive system.

### BACKGROUND

Anchors and their associated systems provide useful fixation devices for tissue repair, such as for repairing tendons or ligament to bone. Anchors may also be used to augment a repair, such as systems disclosed in U.S. Patent Application 17/470,509 titled "JOINT REPAIR AUGMENTATION", commonly owned and herein incorporated by reference in its entirety. Anchors may be inserted using an anchor inserter, either by pounding the anchor into the bone with or without a prepared bone hole. The anchor can be configured as a screw mechanism or an interference fit device and may be made of metal, plastic or bioabsorbable material (which dissolves in the body overtime).

During operation of the anchor, a portion of the anchor may be rotated, and therefore torsional forces may be applied to the anchor via the insertion instrument. For example, the anchor may include a threaded external surface and insertion may include rotatingly advancing the anchor into the tissue, like a screw-type anchor, known in the art. In another example, an anchor may include a means of trapping a suture coupled thereto, and thereby knotlessly locking a suture with an anchor. The term suture may include equivalent flexible members such as suture tape, wire, cable, ribbon or grafts. Knotlessly locking the suture may include axially advancing a threaded plug along an anchor body lumen to trap the suture between the plug and anchor body lumen. Axially advancing the plug may include holding the anchor body stationary, while rotating the plug relative to the anchor body. Stated another way, axially advancing the plug may include rotating the plug while holding the anchor in a manner that resists concomitant rotation.

Means of applying a torsional force to a portion of the anchor, either to rotate the anchor itself or resist rotation while other elements are rotated may add features and size to the anchor body and associated system. An example existing anchor system is disclosed in U.S. Patent 10,617,406, commonly owned and herein incorporated by reference. This existing anchor systems add a shaped bore in the proximal end of the anchor, that may be square shaped. A shaped bore may receive a mating inserter end therein with a corresponding cross section. In this example the bore inner diameter engages and surrounds the outer peripheral surface of the mating inserter end. In this example anchor, torsional forces are applied to the anchor to advance a plug into the distal eyelet, while the anchor body is held stationary. A shaped anchor bore, and inserter distal end together have an inherent volume of material and therefore may add profile or size to the anchor. A larger anchor may require excessive removal of bone tissue, which in some parts of the body, such as the extremities, may not always be available. Non-limiting example bones of the extremities include bones in the foot, ankle, wrist or hand. Therefore, there is a need to provide an anchor with a transmission mechanism that may apply a torsional force to a portion of an anchor, that has a minimal profile. There is a need for a transmission mechanism for small knotless anchor systems, and thereby small in profile, that allows torsional forces to be applied to the anchor to keep the anchor stationary, while allowing for rotation of a knotless mechanism to advance through the transmission mechanism.

### SUMMARY

Described herein is a knotless anchor system that is streamlined to reduce the profile of the knotless anchor system, which may be particularly advantageous where space or target tissue is limited. Knotless anchor system may include an anchor body, an inserter and a plug spanning therebetween. Inserter and anchor body are coupled in a low-profile manner to insert the anchor body into a tissue, and rotationally stabilize the anchor body while advancing the plug. The anchor system provides a transmission mechanism for small knotless anchors that incorporates a puzzle cut to rotationally stabilize the anchor body while allowing torsional forces to be applied to a plug, adding minimal profile or outermost diameter of the anchor body. The transmission mechanism includes an inserter sleeve and anchor body that is axially coincident and each share both an outer diameter and inner diameter along the transmission mechanism.

Disclosed herein is an example suture anchor system that includes an anchor body, a plug and an insertion instrument. The anchor body is configured to anchor within a target tissue and may include externally disposed bone engaging members. The anchor body generally includes a proximal portion, a distal tip that may be tapered and an outer surface extending therebetween. The anchor body may be a rigid tubular body formed of a metal or polymer material. The anchor body may be formed of PEEK. The anchor body may include an eyelet extending therethrough for receiving at least one flexible member such as a suture therethrough. The anchor body also includes a cavity extending axially from the eyelet and up to and including the proximal open end of the anchor body. The anchor body also includes an annular projection extending from the proximal end defining a lumen continuous and coaxial with the cavity, the annular projection having an annular slot extending axially therealong. The plug is configured to move axially along both the anchor body cavity and annular projection lumen and moves to knotlessly lock the flexible member that extends through the eyelet with the anchor body. The insertion instrument may have a sleeve at the distal end that houses a portion of the plug as the system is obtained. The sleeve has an annular tab extending axially from the sleeve distal end, the tab continuous with the sleeve outer surface. The annular slot of the anchor body is sized and shaped to slidingly receive the annular tab therein to interdigitate when the insertion instrument and anchor body are linked. When coupled in this way this forms an annular connecting portion that includes an inner-lumen surface and an external surface that both include the annular projection and the annular tab. The annular connecting potion allows torsional forces to be applied to the plug to axially advance it, while keeping the anchor body stationary, with minimal added profile to the system.

In some example systems the annular tab has a threaded inner surface for threadingly engaging external threads on the plug. The sleeve distal end may also include internal threads to threadingly engage threads on the plug. The anchor body cavity may also include threads such that, as obtained the plug is threadingly engaged with the anchor body cavity and the sleeve distal end internal threads. This helps retain the anchor plug and instrument coupled during use. In some example systems, the anchor body annular projection is absent threads, and the annular tab inner surface is threaded to also threadingly engage threads of the plug. In this example, the annular connecting portion inner-lumen surface therefore includes a segment that is threaded and a segment that is not threaded. The annular projection may define a first annular segment length, and the annular tab may define a second annular segment length and wherein the first annular segment length may be greater than and may be at least double that of the second annular segment length. The greater first annular segment length may compensate for the weaker or less rigid materials of the anchor body relative to the inserter tabs. In some example embodiments the eyelet may traverse the anchor body, defining an entrance and exit aperture, and the exit and entrance apertures may align with the annular projection so that that suture extends along a channel and over a smooth annular project rather than over the discontinuity of the slot and tabs. The eyelet may traverse the anchor body, defining an entrance and exit aperture, and wherein the exit and entrance apertures may be circumferentially offset from the annular slot.

An example embodiment of a knotless suture anchoring system is disclosed, including an anchor body and an insertion instrument. The anchor body anchor within a target tissue and includes a tubular body, a means of engaging the target tissue and proximal connecting portion for functionally linking with the insertion instrument. The insertion instrument inserts the anchor body within the target tissue and includes a distally disposed instrument connecting portion at a distal end of an insertion instrument sleeve. The anchor body connecting portion and the insertion instrument connecting portion are configured to interlink or assemble such that they axially overlap and interdigitate to form a tubular connecting portion with a shared inner diameter and a shared outer diameter.

In some example embodiments, the system also includes a plug, that is moved axially along a cavity of the anchor body via actuation means of the insertion instrument. The plug also axially advances along a tubular connecting portion lumen defined by the shared inner diameter. The plug moves into the anchor body to knotlessly lock a suture or equivalent flexible member with the anchor body. The plug may be rotated while moving axially and the tubular connecting portion is configured to inhibit anchor body rotation while rotating the plug. The anchor body proximal connecting portion may define an annular projection with two annular slots therethrough and the insertion instrument connecting portion may include two annular tabbed projections on opposing sides to each other of the sleeve, the two annular tabbed projections configured to be received by, one each, the two annular slots to define the tubular connecting portion. The instrument connecting portion may be threaded to threadingly engage threads of the plug and the anchor body proximal connecting portion may be absent threads, so that the shared inner diameter of the tubular connecting portion includes a segment that is threaded and a segment that is absent threads. The proximal connecting portion may define a first circumferential segment length of the tubular connecting portion and the instrument connecting portion defines a second circumferential segment length of the tubular connecting portion and wherein the first circumferential segment length is greater than the second circumferential segment length. The anchor body may also include an eyelet through the anchor body, having an entrance and exit aperture for receiving a suture therethrough, and the exit and entrance apertures may be circumferentially offset from the two annular slots of the anchor body connecting portion.

An example method of knotless locking a tissue repair is also disclosed, including obtain a suture anchor system as disclosed herein. A suture may then be inserted through the eyelet and then tensioned to a target tension. The plug may be rotated to axially advance it through the annular connecting portion and into the anchor body cavity and while rotating, the anchor body may be held static via the annular connecting portion. A distal terminus of the plug may engage with the suture to knotless locking the suture.

In some example methods, the plug may be threaded, and a portion of the annular connecting portion may also be threaded to threadingly engage the plug. The insertion instrument may be removed after the suture has been knotlessly locked. Inserting the suture may also include extending the suture from the eyelet along the anchor body and along the annular projection, at a location circumferentially spaced away from the annular slot.

These and other features and advantages will be apparent from a reading of the following detailed description and a review of the associated drawings. It is to be understood that both the foregoing general description and the following detailed description are explanatory only and are not restrictive of aspects as claimed.

### BRIEF DESCRIPTION OF THE DRAWINGS

The disclosure will be more fully understood by reference to the detailed description, in conjunction with the following figures, wherein:
FIG. 1 illustrates a knotless anchor system with a puzzle cut connecting portion, in accordance with this disclosure;
FIG. 2 illustrates a distal end of the knotless anchor system with a puzzle cut connecting portion, in accordance with this disclosure;
FIG. 3A illustrates a perspective view of an anchor body of the knotless anchor system of this disclosure;
FIG. 3B illustrates a side view of the anchor body of the knotless anchor system of this disclosure;
FIG. 3C illustrates another side view of the anchor body of the knotless anchor system of this disclosure;
FIG. 3D illustrates a cross section view of the anchor body of the knotless anchor system of this disclosure;
FIG. 4 illustrates an exploded view of the anchor body and inserter of the knotless anchor system, with a plug and driver removed, in accordance with this disclosure;
FIGS. 5A and 5B illustrate cross sections of the distal end of the knotless anchor system with a puzzle cut connecting portion, in accordance with this disclosure;
FIG. 6 illustrates a cross section through the puzzle cut connecting portion of the knotless anchor system, in accordance with this disclosure; and
FIGS. 7A and 7B illustrate various views of another example embodiment of an anchor body of a knotless anchor system with a puzzle cut connecting portion.

### DETAILED DESCRIPTION

In the description that follows, like components have been given the same reference numerals, regardless of whether they are shown in different examples. To illustrate example(s) in a clear and concise manner, the drawings may not necessarily be to scale and certain features may be shown in somewhat schematic form. Features that are described and/or illustrated with respect to one example may be used in the same way or in a similar way in one or more other examples and/or in combination with or instead of the features of the other examples.

As used in the specification and claims, for the purposes of describing and defining the invention, the terms "about" and "substantially" are used to represent the inherent degree of uncertainty that may be attributed to any quantitative comparison, value, measurement, or other representation. The terms "about" and "substantially" are also used herein to represent the degree by which a quantitative representation may vary from a stated reference without resulting in a change in the basic function of the subject matter at issue. "Comprise," "include," and/or plural forms of each are open ended and include the listed parts and can include additional parts that are not listed. "And/or" is open-ended and includes one or more of the listed parts and combinations of the listed parts. Use of the terms "upper," "lower," "upwards," and the like is intended only to help in the clear description of the present disclosure and are not intended to limit the structure, positioning and/or operation of the disclosure in any manner.

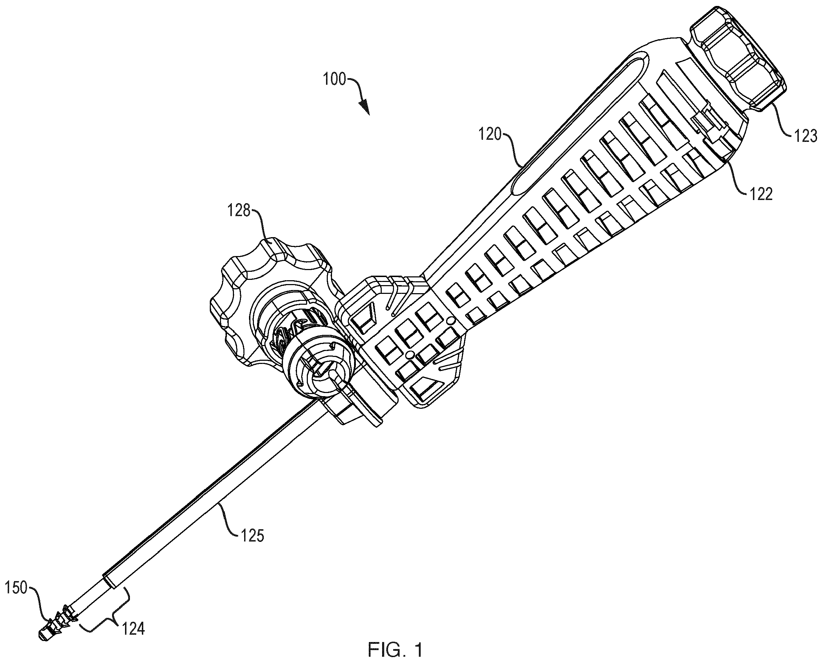

FIG. 1 illustrates an anchoring system 100 including an inserter 120 and anchor body 150. Inserter 120 may include a handle 122 at a proximal end with actuator 123, a shaft 125 and shaft distal end 124. Anchor body 150 is coupled to inserter 120 at distal end 124. In some embodiments, a suture (or equivalent flexible member) may be coupled to the anchor body 150 and then to a tensioning member 128 for controlling a tension on the suture. This may be used when system 100 is part of a ligament augmentation system, for example, similar to that disclosed in commonly owned Patent Application No. 17/470509 titled "JOINT REPAIR AUGMENTATION", herein incorporated by reference.

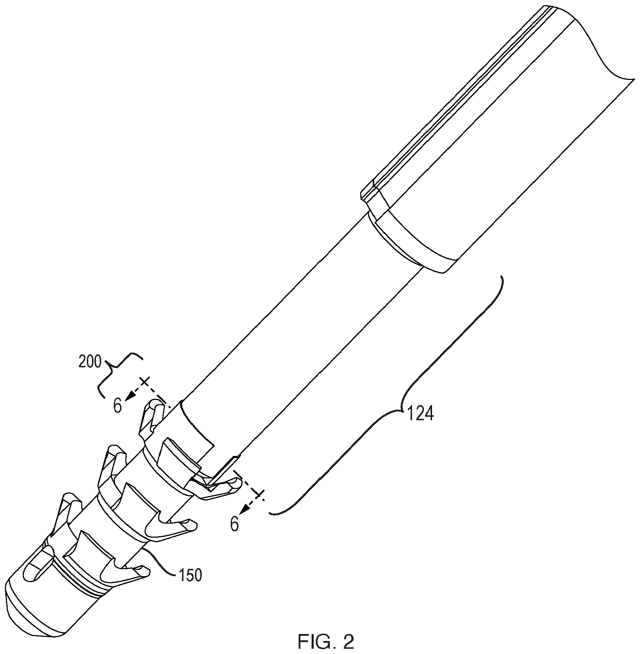

FIG. 2 illustrates the anchor body 150 coupled to inserter distal end 124 defining a connecting portion 200. Connecting portion 200 includes axially overlapping portions of both the inserter shaft distal end 124 and anchor body proximal end in a circumferentially arranged finger-joint style interlock.

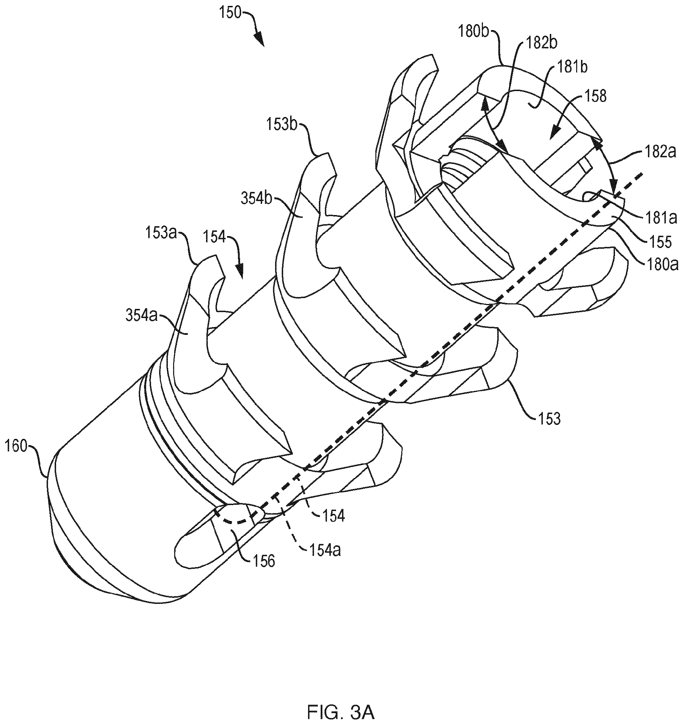

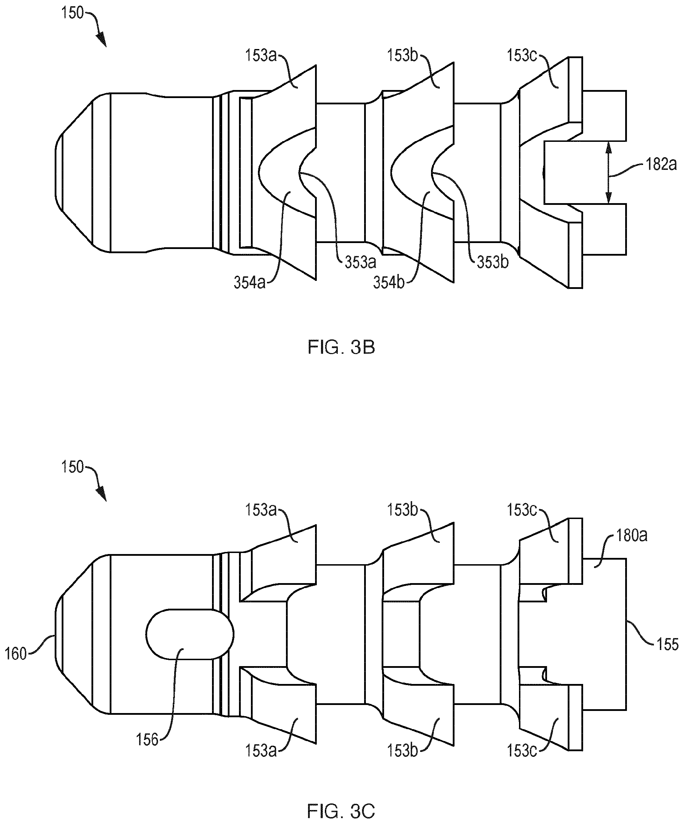

FIGS. 3A-3D illustrate a variety of views of anchor body 150. Anchor body 150 may be an elongate tubular body, with a proximal end 155 and a closed tapered distal end 160. The anchor body 150 may also have an eyelet 156, extending transversely through a longitudinal axis of the anchor body 150, and dimensioned to receive one or more sutures (or equivalent) therethrough (sutures not shown). The anchor body 150 further includes a cannulation 158 formed within the anchor body 150 and intersecting the eyelet 156. Anchor body 150 includes at least one bone engaging member 153, extending radially from the body 150. As illustrated, anchor body 150 may include two rows of axially spaced bone engaging members 153, the two rows extending from opposite sides of the body 150 from each other. The bone engaging members 153 may be barbs or wings for example. An external channel 154 may extend axially along an outer surface of body 150 from eyelet 156 to proximal end 155, for guiding and receiving a length of suture therealong. The external channel 154 may define a circumferential spacing between the two rows of axially spaced bone engaging members 153. A path 154a is shown illustrating a suture path from eyelet 156 along external channel 154. A second channel and corresponding path that may be diametrically opposite path 154a may also extend along the body 150 on the opposing side.

Proximal end 155 of anchor body 150 defines an anchor portion of the connecting portion 200. Proximal end 155 includes two axially extending prongs 180a, 180b. Connecting portion 200 may define circumferentially arranged finger-joint style interlock, to define a cylindrical shape when the inserter and anchor body are coupled. Prongs 180a, 180b define annular segments of cylindrical shaped connecting portion 200 (transmission mechanism) between the anchor body 150 and inserter distal end 124. An axially moving plug (shown later) is disposed within and along connecting portion 200 and also within cannulation 158. Prongs 180a, 180b may include smooth inner diameter walls 181a, 181b. Prongs 180a, 180b may extend axially and circumferentially and define annular gaps 182a, 182b that are configured to receive axially extending prongs of shaft distal end 124 (shown later). Prongs 180a, 180b may define annular gaps 182a, 182b, the gaps circumferentially offset from channels 154. Gaps 182a, 182b may be axially aligned with the rows of axially spaced bone engaging members 153. Prongs 180a, 180b may be on the same circumferential side and axially continuous with eyelet opening and channel(s) 154, such that a suture may extend from the eyelet 156 axially along channel(s) 154 linearly along and parallel to the anchor body longitudinal axis, and also over a smooth outer surface of a prong (180a, 180b). Circumferential ends of the bone engaging members 153 may help retain suture within the channel(s) 154.

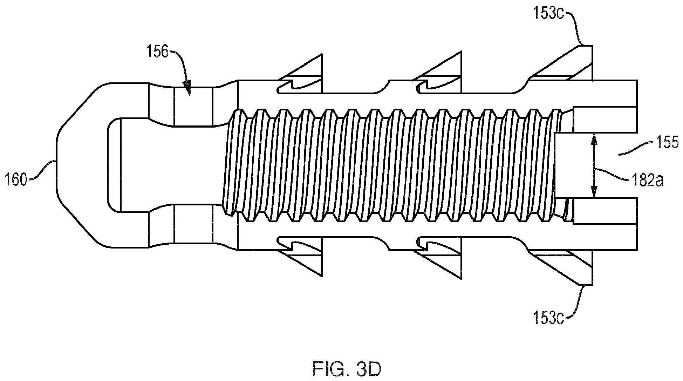

Illustrated in FIG. 3B is one of the rows of axially spaced bone engaging members 153, including members 153a, 153b and 153c. At least some of the members 153a, 153b may include a concave recess 353a, 353b, that is arranged on a proximal edge of the member and also centered along each of the bone engaging member 153a, 153b. The recess 353a, 353b may improve flexibility of the member 153a, 153b as they flex during insertion into the target bone. Concave recess 353a, 353b may be tapered along a length of each bone engaging member, to define a smooth contoured concave external surface 354a, 354b. Concave recesses 353a, 353b may axially align with one of the annular gaps 182a or 182b. Also shown in FIG. 3B, a proximal most bone engaging member 153c may be split, the split extending circumferentially and approximately matching a circumferential extent of one of the adjacent annular gaps (182a, 182b). This proximal most bone engaging member 153c may be larger than the other members 153a, 153b, in both volume and radial extent (also shown in FIG. 3D). The proximal most bone engaging member 153c may provide the strongest fixation. The added volume may increase rigidity of the member 153c and the radial extension may encroach further into the bone tissue.

FIG. 3D illustrates a cross sectional views of anchor 150. Cannulation 158 may include internal threads therealong, for threadingly engaging the plug.

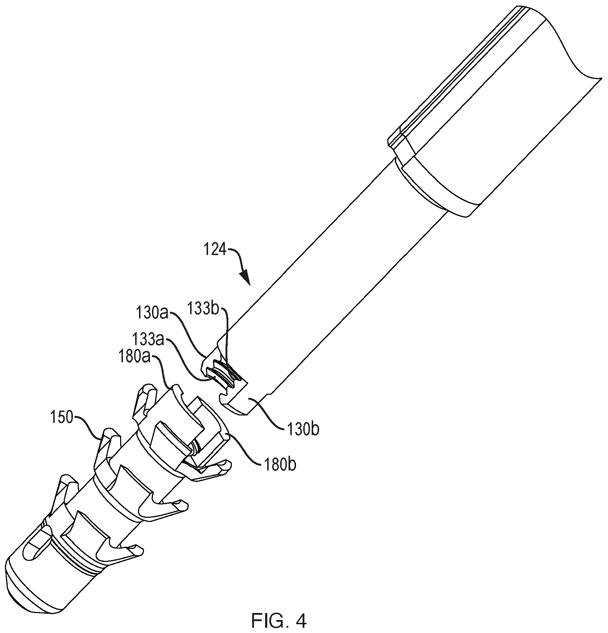

FIG. 4 illustrates an exploded view of the inserter distal end 124 and anchor body 150, with the plug and plug driver removed for clarity of the figure. Prongs 180a, 180b define annular gaps 182a, 182b, for receipt of inserter tabs 130a, 130b therein. This puzzle cut coupling rotationally stabilizes the anchor body 150 relative to the inserter 120, while keeping a low profile. When prongs 180a, 180b interdigitate with tabs 130a, 130b, they form a tubular construct that shares both an outer and inner diameter therealong. The outer circumferential surface of the resulting tubular interlinking joint (connecting portion 200) is formed by both the prongs 180a, 180b and tabs 130a, 130b. The inner circumferential surface of the resulting tubular interlinking joint (connecting portion 200) is formed by both the prongs 180a, 180b and tabs 130a, 130b. Inserter tabs 130a, 130b may define a threaded inner diameter surface 133a, 133b that threadingly engages a plug. Prongs 180a, 180b and tabs 130a, 130b may have an axial length sufficient to counter any rotation and stably hold the anchor body. Prongs 180a, 180b and tabs 130a, 130b may be between 2-5mm in length. While two prongs and tabs are shown, circumferentially arranged symmetrically around the connecting portion 200, other numbers are envisioned. In some embodiments there may be only one prong and one tab. In other embodiments there may be three of each, circumferentially arranged relative to each other.

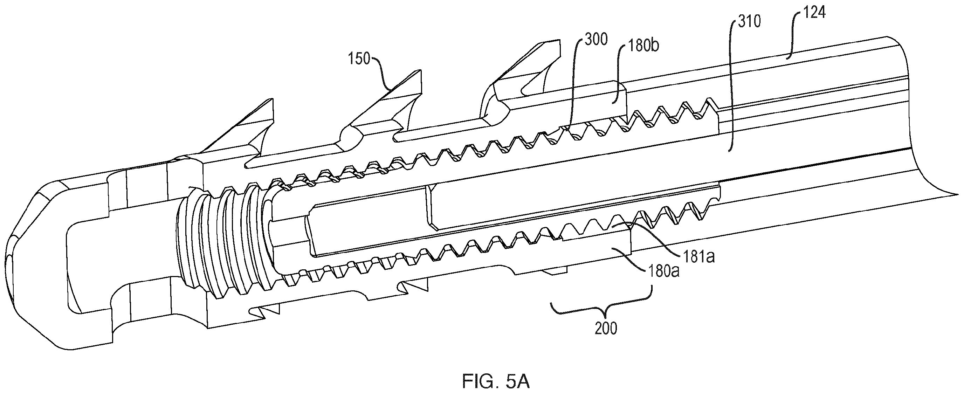

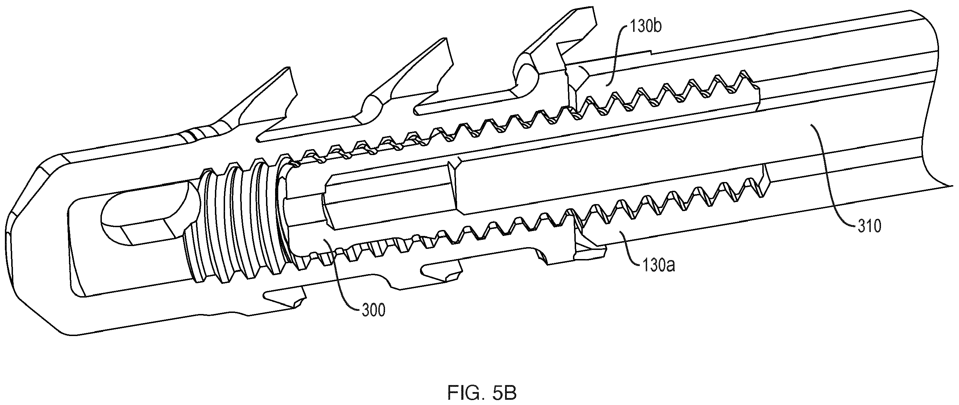

FIG. 5A and 5B illustrate longitudinal cross sections of the system distal end, including anchor body 150, plug 300, plug actuation rod (plug driver) 310 and inserter distal end 124. The plug 300 may be a generally elongated, tubular threaded rod with a cannulation adapted to receive a distal end of the rod 310. Plug 300 and rod 310 may be similar to and be operated in a similar fashion the commonly owned US Patent 10,617,406, herein incorporated in its entirety by reference. Axially advancing plug, via actuation of rod 310 may advance the plug 300 to as to engage and knotlessly lock a suture extending through eyelet 156.

FIG. 5A and 5B both illustrate the assembly without a suture. In both FIG. 5A and 5B, the plug 300 is also shown in a proximal location, threadingly coupled to both the anchor body 150 and inserter distal end 124. This provides stability while inserting the anchor 150 into the tissue. The system 100 may be provided with the plug 300 in this location. As provided the plug 300 is proximally spaced from eyelet 156 so that any suture or equivalent flexible member may be threaded through and slid through eyelet 156 of anchor body 150. Plug 300 may be axially advanced to knotless lock the suture (not shown) via rod 310. Rod 310 may be operatively coupled to an actuator 123 on the handle 122, and rotation of rod 310 may axially advance the plug 300 while the connection portion 200 holds the anchor body 150 stationary. In FIG. 5A the connecting portion 200 is shown with a cross section that includes only the anchor prongs 180a, 180b. Prongs 180a, 180b may have a smooth (non-threaded) inner circumferential surface and may define an inner diameter than provide clearance for the threaded plug 300 to axially advance through. In FIG. 5B the cross section shown extends through the inserter sleeve tabs 130a, 130b. Tabs 130a, 130b may define a threaded inner surface, that threadingly engages the threaded plug 300. Therefore, the inner circumferential surface of the connecting portion 200 may include at least one annular segment that is threaded and at least one segment that is non-threaded.

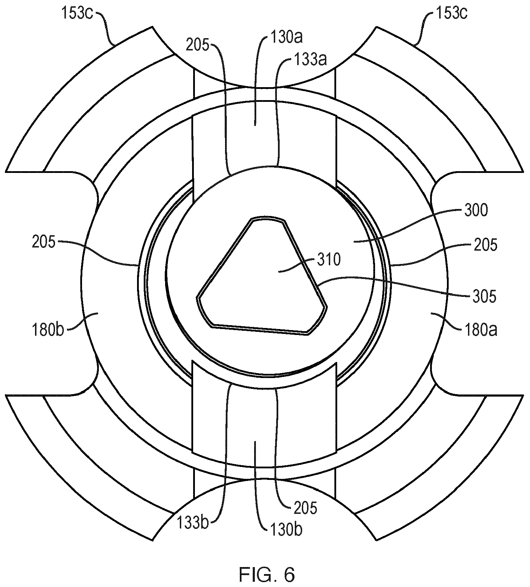

FIG. 6 illustrates a transverse cross section of the connecting portion 200, at the location shown in FIG 2 (illustrated with the number "6"). Threaded plug 300 is shown with rod 310. Plug cannulation 305 may be shaped to receive a rod 310, that may be configured to rotate plug 300. Cannulation 305 and rod cross section may have a cross section that is triangular as shown. Other non-limited examples may be square, rectangular or hexagonal. Cross section of connecting portion 200 is shown, including both anchor prongs 180a, 180b and inserter tabs 130a, 130b. All prongs and tabs may be curved to define an approximate tubular shape along the connecting portion 200, as interlinked. The discontinuities are due to internal threads on the tabs 130a, 130b. Connecting portion 200, when interlinked defines an inner diameter surface 205 that may include discontinuities due to threads on the tabs 130a, 130b. A least a portion of inner diameter surface 205 may engage plug 300, such as threaded inner surfaces 133a of inserter tab 130a. In other embodiments inner diameter surface 133a, 133b may be smooth (non threaded). Inserter prongs 130a, 130b may therefore extend slightly further radially inward, to accommodate the threads. Anchor body prongs 180a, 180b may define longer segment lengths than tabs 130a,130b, which may balance the offset in materials and relative structural integrity between the anchor body 150 and inserter distal end 124. Anchor body 150 may be fabricated from a polymer that may be bioabsorbable, while the anchor inserter distal end 124 may be include metal, and therefore inherently a mismatch in structural integrity may exist therebetween. In cross section, therefore, the annular cross section may define a 360-degree bounded cannulation when interlinked. The boundary of the 360-degree bounded cannulation may include an annular segment or plurality of annular segments formed by the anchor prongs 180a, 180b, and an annular segment or plurality of annular segments formed by the inserter tabs 130a, 130b. In some embodiments the annular segment or plurality thereof formed by the anchor prongs 180a, 180b may define substantially more of the 360-degree boundary than the annular segment or plurality thereof formed by the inserter tabs 130a, 130b. For example, in some embodiments the segment or plurality of segments formed by the anchor prongs 180a, 180b may define at least double the boundary length relative to the segment or plurality of segments formed by the inserter tabs 130a, 130b. For example, in some embodiments the segment or plurality of segments formed by the anchor prongs 180a, 180b may define double the boundary length relative to the segment or plurality of segments formed by the inserter tabs 130a, 130b.

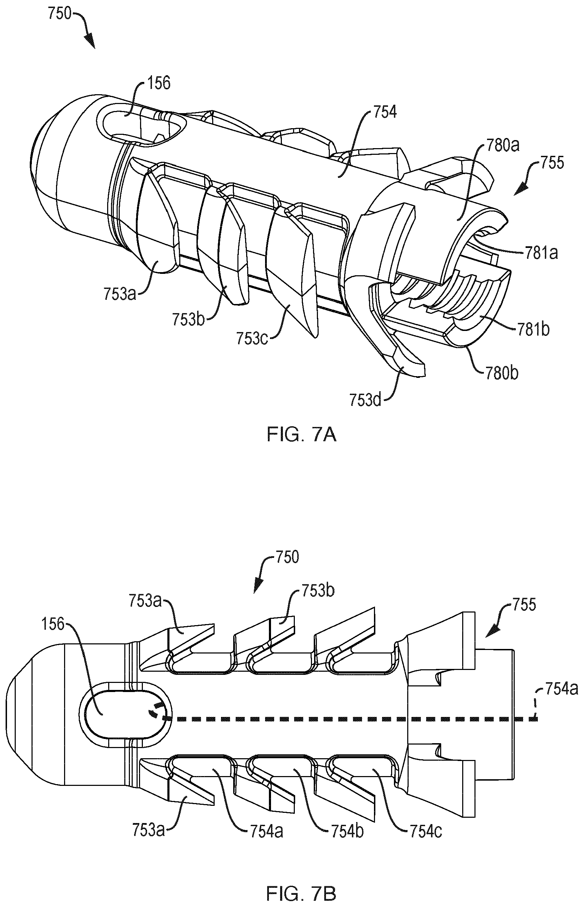

FIGS. 7A and 7B illustrates another example embodiment of an anchor body 750, anchor body 750 like anchor body 150 except where described. Same or essentially similar elements are given the same label numbers. Anchor body 750 may couple to inserter 120 at shaft distal end 124. A suture (or equivalent flexible member) may be coupled to the anchor body 750 and then to a tensioning member 128 for controlling a tension on the suture. Anchor body 750 may couple to inserter distal end 124 defining a connecting portion similar to that shown in FIG. 2. Connecting portion includes axially overlapping portions of both the inserter shaft distal end 124 and anchor body proximal end 755 in a circumferentially arranged finger-joint style interlock.

Anchor body 750 may be an elongate tubular body, with a proximal end 755 and a closed tapered distal end 160. The anchor body 750 may also include eyelet 156 and cannulation 158 formed within the anchor body 750 and intersecting the eyelet 156. Anchor body 750 includes at least one bone engaging member 753a, 753b, 753c, 753d, extending radially from the body 750. As illustrated, anchor body 750 may include two rows of axially spaced bone engaging members, the two rows extending from opposite sides of the body 750 from each other. The bone engaging members may be barbs or wings for example. An external surface 754 may extend axially along an outer surface of body 750 from eyelet 156 to proximal end 755, for guiding and receiving a length of suture therealong. The external channel 754 may define a circumferential spacing between the two rows of axially spaced bone engaging members 753a, 753b, 753c, 753d. A path 754a is shown illustrating a suture path from eyelet 756 along external channel 754. A second channel and corresponding path that may be diametrically opposite path 754a may also extend along the body 750 on the opposing side.

Proximal end 755 of anchor body 750 defines an anchor portion of connecting portion that is similar to connecting portion, 200 except as noted. Proximal end 755 includes two axially extending prongs 780a, 780b, similar to prongs 180a, 180a, in that they define circumferentially arranged finger-joint style interlock, to define a cylindrical shape when the inserter and anchor body are coupled. Prongs 780a, 780b are similar to prongs 180a, 180a except that they include internal threaded surfaces 781a, 781b. As obtained, plug may be disposed along connecting portion 200 including threadingly engaging prong threaded surfaces 781a, 781b. Prong threaded surfaces 781a, 781b may define continuous threads with threaded inner lumen surface of cavity 158, such that external threads of plug 300 threadingly engage both threaded surface 781a, 781b and also the threaded inner lumen surface of cavity 158 as obtained. A threaded engagement between plug 300 and surfaces 781a, 781b may improve the general stability and hold between the inserter 120, plug 300 and anchor 750 during insertion and general manipulation of the system

Illustrated in both FIG. 7A and FIG. 7B each row of axially spaced bone engaging members may include four members 753a, 753b, 753c and 753d. Each of the four members 753a, 753b, 753c and 753d may define radially projecting members that are different from each other. The distal most member 753a may be the smallest and most tapered. The distal most member 753a may extend the least radially from the body 750 relative to the other members, 753b, 753c and 753c. Each member 753a, 753b, 753c and 753d may increase in both rigidity and radial extension the further proximally disposed the member is along the row. Each member 753a, 753b, 753c and 753d may define a projection that is different from the others. This may provide for easier insertion, as the distal most member 753a, the most flexible and radially closest of the members enters the bone opening first, the insertion force, and thereby fixation force gradually increasing as each successive member (753b, 753c, 753d) enters the bone opening.

Anchor body 750 may also include rows of external cavities 754a, 754b, 754c, directly adjacent a corresponding member 753a, 753b and 753c, as clearance for members 753a, 753b and 753c as they flex during anchor insertion. Cavities 754a, 754b, 754c may therefore be sized to receive a portion of their corresponding fixation member 753a, 753b and 753c.

A method of knotless securing a suture (or equivalent) within bone tissue may include obtaining system 100, the system 100 including an inserter 120 and an anchor body (150 or 750) with an eyelet 156 extending therethrough. At least one suture may be obtained and threaded through the eyelet 156. The suture may then be tensioned. The suture may be operatively coupled to a tensioning wheel 128 of the inserter 200 to tension the suture. The system 100 may be obtained with a threaded tubular plug 300 threadingly coupled to both the anchor body (150 or 750) and inserter 120. The threaded plug may extend and directly threadingly engage a threaded cannulation 158 of the anchor body 150 or 750 and a threaded cannulation of the inserter distal end 124. In this location, the threaded plug 300 may extending along a lumen of a connecting portion 200, the lumen boundary shared by both the anchor body (150, 750) and inserter distal end 124. The connection portion may include an annular segment that includes threads, to threadingly engage the plug 300.

Once the desired tension has been achieved on the suture, the threaded plug 300 may be axially advanced further into the anchor body (150, 750) to knotlessly lock the suture with the anchor body (150, 750). Axially advancing may include rotating the plug 300 while holding the anchor body (150, 750) stationary. The connecting portion defines an annular interlinking joint that holds the anchor body stationary while the plug rotates. A distal terminus of the plug 300 may move from a first axial position to a second axial position; wherein, in the first axial position, the plug 300 engages both the anchor body (150, 750) and the threaded distal end of the inserter 120 and a distal terminus of the plug 300 is proximal to the eyelet; and wherein, while moving to the second axial position, the plug advances further into the anchor body (150, 750) threadingly disengages from the inserter 120 and may also covers at least a portion of the eyelet 156 so as to secure the suture between a distal terminus of the plug 300 and distal bottom end of the anchor cannulation 158. In the second axial position, the plug 300 may be entirely recessed from the body proximal end 155, 755. The inserter may now be removed, leaving the plug and anchor within the bone tissue.

One skilled in the art will realize the disclosure may be embodied in other specific forms without departing from the spirit or essential characteristics thereof. The foregoing examples are therefore to be considered in all respects illustrative rather than limiting of the disclosure described herein. Scope of the disclosure is thus indicated by the appended claims, rather than by the foregoing description, and all changes that come within the meaning and range of equivalency of the claims are therefore intended to be embraced therein.

Attention is directed to all papers and documents which are filed concurrently with or previous to this specification in connection with this application and which are open to public inspection with this specification, and the contents of all such papers and documents are incorporated herein by reference.

All of the features disclosed in this specification (including any accompanying claims, abstract and drawings), and/or all of the steps of any method or process so disclosed, may be combined in any combination, except combinations where at least some of such features and/or steps are mutually exclusive.

Each feature disclosed in this specification (including any accompanying claims, abstract and drawings) may be replaced by alternative features serving the same, equivalent or similar purpose, unless expressly stated otherwise. Thus, unless expressly stated otherwise, each feature disclosed is one example only of a generic series of equivalent or similar features.

The invention is not restricted to the details of the foregoing embodiment(s). The invention extends to any novel one, or any novel combination, of the features disclosed in this specification (including any accompanying claims, abstract and drawings), or to any novel one, or any novel combination, of the steps of any method or process so disclosed.

## Claims

1. A suture anchor system comprising:
an anchor body configured to anchor within a target tissue, the anchor body comprising;
a proximal portion, a distal tip and an outer surface extending therebetween;
an eyelet extending through the anchor body;
a cavity extending from the eyelet, axially up to and including the proximal end of the anchor body;
an annular projection extending from the proximal end defining a lumen continuous with the cavity, the annular projection having an annular slot extending axially therealong;
a plug, configured to move axially along both the anchor body cavity and annular projection lumen, wherein when a suture is disposed through the eyelet, the plug is configured to move axially to knotlessly lock the suture with the anchor body; and
an insertion instrument having a sleeve configured to house a portion of the plug, the sleeve having an annular tab extending axially from a sleeve distal end, wherein the annular slot is configured to receive the annular tab therein to interdigitate when the insertion instrument and anchor body are linked to form an annular connecting portion, the annular connecting portion including an inner-lumen surface and an external surface that both include both the annular projection and the annular tab.

2. The suture anchor system of claim 1 wherein the annular tab has a threaded inner surface for threadingly engaging external threads on the plug.

3. The suture anchor system of claim 1 wherein the sleeve distal end includes internal threads to threadingly engage threads on the plug.

4. The suture anchor system of claim 1 wherein the anchor body annular projection is absent threads.

5. The suture anchor system of claim 4 wherein the plug includes a threaded outer surface and wherein the annular tab inner surface is threaded to threadingly engage the plug threaded outer surface, so that the annular connecting portion inner-lumen surface includes a segment that is threaded and a segment that is not threaded.

6. The suture anchor system of claim 1 wherein the annular projection defines a first annular segment length, and the annular tab defines a second annular segment length and wherein the first annular segment length is at least double that of the second annular segment length.

7. The suture anchor system of claim 1 wherein the eyelet traverses the anchor body, defining an entrance and exit aperture, and wherein the exit and entrance apertures align with the annular projection.

8. The suture anchor system of claim 1 wherein the eyelet traverses the anchor body, defining an entrance and exit aperture, and wherein the exit and entrance apertures are circumferentially offset from the annular slot.

9. A suture anchor system comprising:
an anchor body for anchoring within a target tissue, the anchor body comprising a proximal connecting portion; and
an insertion instrument for inserting the anchor body within the target tissue, the insertion instrument having an instrument connecting portion at a distal end of an insertion instrument sleeve;
wherein the anchor body connecting portion and the insertion instrument connecting portion are configured to assemble to each other such that they axially overlap and interdigitate to form a tubular connecting portion with a shared inner diameter and a shared outer diameter.

10. The suture anchor system of claim 9 further comprising a plug, configured to move axially along a cannulation of the anchor body and knotlessly lock a suture with the anchor body and wherein the tubular connection portion shared inner diameter is continuous with the cannulation is it also configured to receive the plug therein.

11. The suture anchor system of claim 10 wherein the plug is configured to rotate while moving axially along the cannulation and wherein the tubular connecting portion is configured to inhibit the anchor body from rotating while rotating the plug.

12. The suture anchor system of claim 9 wherein the anchor body proximal connecting portion defines an annular projection with two annular slots therethrough and the insertion instrument connecting portion includes two annular tabbed projections circumferentially spaced from each other, the two annular tabbed projections configured to be received by, one each, the two annular slots to define the tubular connecting portion.

13. The suture anchor system of claim 10 wherein the instrument connecting portion includes internal threads, configured to threadingly engage threads of the plug and the anchor body proximal connecting portion is absent threads, so that the shared inner diameter of the tubular connecting portion includes a segment that is threaded and a segment that is absent threads.

14. The suture anchor system of claim 9 wherein the proximal connecting portion defines a first circumferential segment length of the tubular connecting portion and the instrument connecting portion defines a second circumferential segment length of the tubular connecting portion and wherein the first circumferential segment length is greater than the second circumferential segment length.

15. The suture anchor system of claim 12 wherein the anchor body further comprises an eyelet through the anchor body, having an entrance and exit aperture for receiving a suture therethrough, and wherein the exit and entrance apertures are circumferentially offset from the two annular slots of the anchor body connecting portion.
